# EUROPEAN PATENT APPLICATION

(11) **EP 4 344 640 A1**
(43) Date of publication of application: **03.04.2024**
(21) Application number: 22198537.7
(22) Date of filing: 28.09.2022
(51) Int. Cl.: A61B 5/369, A61B 5/00, A61B 5/11, A61B 5/0537

(54) **APPARATUS AND METHOD FOR CONTROLLING AN ELECTROENCEPHALOGRAPHY MEASUREMENT**

(71) Applicant: Brainhero GmbH, 1090 Wien (AT)
(72) Inventor: POLLREISZ, David, 1230 Wien (AT); GÖTZ, Christof, 1080 Wien (AT); DELIU, Elena, 1040 Wien (AT)
(74) Representative: 2SPL Patentanwälte PartG mbB

(57) **Abstract**

An apparatus 30 for controlling an electroencephalography, EEG, measurement, comprises interface circuitry 32 configured to receive an input signal comprising information indicative of a behavior of a user based on an output signal of a sensor. Further, the apparatus 30 comprises processing circuitry 34 configured to detect a possible disturbance of the EEG measurement based on the received input signal and to trigger an output to the user to inform the user about the possible disturbance of the EEG measurement.

## Description

### Background

The human brain waves provide an electrical signal at the level of the scalp within the microvolt range. By connecting removable electrode leads to the head, e.g., the scalp, of the subject, those faint electrical signals can be detected and amplified. In the past, those electrical signals were amplified and recorded on a paper strip chart showing an analog wavy line for each recording channel in an EEG (electroencephalograph). EEG has been used for many medical and testing purposes, for example, the testing of auditory, visual, somatosensory and motor systems and testing for pathological brain dysfunction. Presently there are available EEG instruments which can simultaneously detect the faint signals and convert the signals to digital data for recording and analysis.

However, a major problem in obtaining an accurate recording of brain waves has been "movement artifact", which consists of electrical contaminants arising as a consequence of the subject's muscle movements. For example, when a subject moves her head, the muscle action may result in a contaminating electrical signal that will distort or drown out the brain signal. Movement artifacts are caused by the relative movement of the electrode with respect to the scalp. The changes in contact of the electrode on the head result in changes in the impedance and induced potentials at the electrode-scalp interface, producing movement artifacts in the measured EEG. Also head movements can cause the electrode wires to generate a current as they move through the ambient 60 Hz magnetic field, producing "electrode sway artifact".

In addition to movement artifacts, "eye artifact" is another source of electrical contaminants in EEG recordings. When the eyes move or the eyelids blink, electrical potentials can be recorded from electrodes located near the eyes: the "electro-oculogram" or EOG. The eye related potential can also be picked up by EEG electrodes resulting in contaminated EEG signals. Sometimes movement and eye artifacts are present in the measured EEG signal simultaneously.

Because of contamination by movement and eye artifacts, subjects must sit still and restrain eye movements during an EEG recording to minimize head, body or eye movement artifacts. Thus, it may be necessary to increase a control of an EEG measurement.

### Summary

Therefore, it is a finding that a control of an EEG measurement can be improved by informing a user about a possible disturbance of the EEG measurement. Thus, the user can take certain measures to counteract the possible disturbance. In this way, control of the EEG measurement can be improved which may increase a quality and/or accuracy of the EEG measurement.

Examples provide an apparatus for controlling an electroencephalography, EEG, measurement comprising interface circuitry and processing circuitry. The interface circuitry is configured to receive an input signal, based on an output signal of a sensor, comprising information indicative of a behavior of a user. The processing circuitry is configured to detect a possible disturbance of the EEG measurement based on the received input signal. Further, the processing circuitry is configured to trigger an output to the user to inform the user about the possible disturbance of the EEG measurement. By using the output signal of the sensor a possible disturbance of the EEG measurement can be detected. Further, by informing the user about the possible disturbance of the EEG measurement the user can take certain measures to prevent and/or to end the possible disturbance. In this way, a quality and/or accuracy of the EEG measurement can be improved

Examples provide an apparatus for controlling an electroencephalography, EEG, measurement comprising interface circuitry and processing circuitry. The interface circuitry is configured to receive an input signal comprising information indicative of a humidity at a head of a user. The input signal is based on an output signal of a humidity sensor of an EEG device. The processing circuitry is configured to determine whether the humidity at the head of the user exceeds a humidity threshold that affects the EEG measurement. Further, the processing circuitry is configured to trigger an output to the user to inform the user about the effect on the EEG measurement, e.g., a possible disturbance of the EEG measurement. By using sensor data of the humidity sensor an effect on the EEG measurement can be determined. The effect on the EEG measurement may disturb the EEG measurement. For example, excessive sweating of a user can substantially change the signal sensor dynamics by facilitating formation of salt bridges and thus affect the quality of the EEG measurement. By informing the user about the effect the user can take certain measures to counteract the effect. In this way, a quality and/or accuracy of the EEG measurement can be improved.

Examples provide an EEG system comprising an apparatus as described above. Further, the EEG system comprises a sensor configured to generate the output signal. In this way, the EEG system can be used to control the EEG measurement by determining sensor data and triggering an output to the user.

### Brief description of the Figures

Some examples of apparatuses and/or methods will be described in the following by way of example only, and with reference to the accompanying figures, in which
Fig. 1 shows a block diagram of an example of an apparatus for controlling an EEG measurement;
Fig. 2 shows an example of a block diagram of an apparatus for controlling an EEG measurement;
Fig. 3 shows an example of an EEG system comprising an apparatus as described in Fig.1 or Fig. 2;
Fig. 4 shows different views of an example of an EEG device comprising an EEG cap and a processing circuitry;
Fig. 5 shows a perspective view of a base station;
Fig. 6 shows an example of a method for controlling an EEG measurement;
Fig. 7 shows another example of a method for controlling an EEG measurement; and
Fig. 8 shows another example of a method for controlling an EEG measurement.

### Detailed Description

Various examples will now be described more fully with reference to the accompanying drawings in which some examples are illustrated. In the figures, the thicknesses of lines, layers and/or regions may be exaggerated for clarity.

Accordingly, while further examples are capable of various modifications and alternative forms, some particular examples thereof are shown in the figures and will subsequently be described in detail. However, this detailed description does not limit further examples to the particular forms described. Further examples may cover all modifications, equivalents, and alternatives falling within the scope of the disclosure. Like numbers refer to like or similar elements throughout the description of the figures, which may be implemented identically or in modified form when compared to one another while providing for the same or a similar functionality.

Some embodiments may have some, all, or none of the features described for other embodiments. "First," "second," "third," and the like describe a common element and indicate different instances of like elements being referred to. Such adjectives do not imply element item so described must be in a given sequence, either temporally or spatially, in ranking, or any other manner. "Connected" may indicate elements are in direct physical or electrical contact with each other and "coupled" may indicate elements co-operate or interact with each other, but they may or may not be in direct physical or electrical contact.

As used herein, the terms "operating", "executing", or "running" as they pertain to software or firmware in relation to a system, device, platform, or resource are used interchangeably and can refer to software or firmware stored in one or more computer-readable storage media accessible by the system, device, platform or resource, even though the instructions contained in the software or firmware are not actively being executed by the system, device, platform, or resource.

The description may use the phrases "in an embodiment," "in embodiments," "in some embodiments," and/or "in various embodiments," each of which may refer to one or more of the same or different embodiments. Furthermore, the terms "comprising," "including," "having," and the like, as used with respect to embodiments of the present disclosure, are synonymous.

It will be understood that when an element is referred to as being "connected" or "coupled" to another element, the elements may be directly connected or coupled or via one or more intervening elements. If two elements A and B are combined using an "or", this is to be understood to disclose all possible combinations, i.e., only A, only B as well as A and B. An alternative wording for the same combinations is "at least one of the group A and B". The same applies for combinations of more than 2 Elements.

The terminology used herein for the purpose of describing particular examples is not intended to be limiting for further examples. Whenever a singular form such as "a," "an" and "the" is used and using only a single element is neither explicitly or implicitly defined as being mandatory, further examples may also use plural elements to implement the same functionality. Likewise, when a functionality is subsequently described as being implemented using multiple elements, further examples may implement the same functionality using a single element or processing entity. It will be further understood that the terms "comprises," "comprising," "includes" and/or "including," when used, specify the presence of the stated features, integers, steps, operations, processes, acts, elements and/or components, but do not preclude the presence or addition of one or more other features, integers, steps, operations, processes, acts, elements, components and/or any group thereof.

Unless otherwise defined, all terms (including technical and scientific terms) are used herein in their ordinary meaning of the art to which the examples belong.

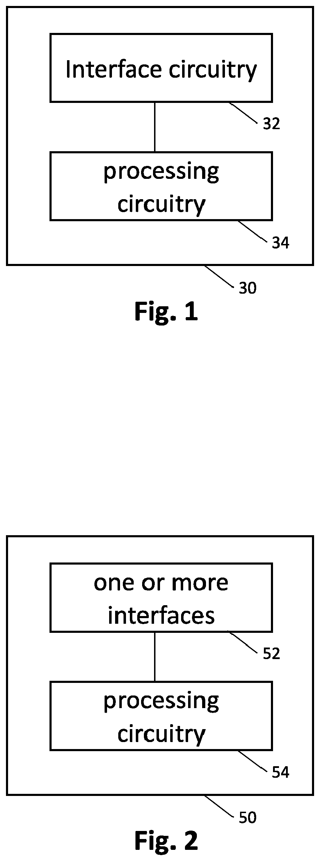

Fig. 1 shows a block diagram of an example of an apparatus 30 for controlling an electroencephalography, EEG, measurement. The apparatus 30 comprises interface circuitry 32 configured to receive an input signal comprising information indicative of a behavior of a user. The input signal is based on an output signal of a sensor. Further, the apparatus 30 comprises processing circuitry 34 configured to detect a possible disturbance of the EEG measurement based on the received input signal. Further, the processing circuitry 34 is configured to trigger an output to the user to inform the user about the possible disturbance of the EEG measurement.

By using sensor data, a possible disturbance of the EEG measurement can be detected, and the user can be informed. By informing the user on a possible disturbance, the user can take countermeasures to reduce or avoid possible disturbances. In this way, the quality and/or accuracy of the EEG measurement can be improved.

The apparatus 30 can be used to obtain data allowing to inform a user about a possible disturbance of the EEG measurement. The apparatus 30 may enable the user to perform the EEG measurement with a desired precision by informing the user about a possible disturbance during the EEG measurement. In this way, a home-based therapy can be performed with clinical grade accuracy, especially in absence of any specialized staff. For example, the apparatus 30 enables a user to run an EEG measurement himself at home.

For example, the apparatus 30 may be part of or connected to an EEG device (e.g., a recording setup). The EEG device may comprise or may be a head-mountable measurement device (e.g., EEG cap or hat) to be worn by a user during an EEG measurement. The head-mountable measurement device may comprise one or more sensor electrodes to measure an EEG signal of the EEG measurement. For example, a user wearing the head-mountable measurement device may be informed about the possible disturbance of the EEG measurement. In this way, the user can be informed about a possible disturbance of his own EEG measurement.

Alternatively, the apparatus 30 may be part of or connected to a user device, e.g., a tablet, a smartphone, a laptop computer, a tablet computer, a personal computer.

For example, the user may control/run the EEG device and/or the EEG measurement with the user device, e.g., via an application executed on the user device. The application can be associated to the EEG device and/or EEG measurement. For example, the user can start the EEG measurement with the user device, e.g., by an input on the user device.

The EEG device and/or the user device may be part of an EEG system. The EEG system may be a mobile system, e.g., for home use. The apparatus 30 can be used to improve a control of an EEG measurement and the measurement can be performed at any place.

The sensor can be any sensor suitable to measure a parameter indicative of a behavior of the user, e.g., a movement (e.g., of a head), a blink rate, a pose and/or a result of a behavior of the user, e.g., a generated sound, a transpiration. For example, the sensor may be an accelerometer, a gyroscope, a magnetometer, e.g., to track a movement of the body of the user, especially of the head, a camera, e.g., to track a blink rate of the user, or a humidity sensor, e.g., to measure a humidity of the skin of the user.

The sensor can be part of the EEG device. For example, the head-mountable measurement device may comprise the sensor, e.g., an accelerometer gyroscope, a magnetometer, a humidity sensor. By arranging the sensor at the cap sensor data related to a movement, or a humidity can be measured with improved accuracy.

Alternatively, the sensor can be part of the user device. For example, the sensor can be or can comprise a camera and/or a microphone of the user device. Using a user device sensor can reduce the cost of implementing the sensor. Further, arranging the sensor in the user device may allow improved determination of a user behavior. For example, a camera of the user device may allow to determine a pose of the head of the user relative to the user device with an increased accuracy. The pose of the head may be indicative of an undesired user behavior (e.g., distraction). In this way, the detection of the possible disturbance of the EEG measurement can be improved.

The output signal of the sensor may comprise data, e.g., the raw data measured by the sensor without further processing of the data. For example, the processing circuitry 34 may be configured to process the data, e.g., to determine the user behavior. Alternatively, the output signal may comprise processed data of the sensor comprising information about the user behavior, e.g., a pose of the user, a movement, a blink rate. For example, the user can be informed by the apparatus 30 about a reason of the possible disturbance of the EEG measurement. The apparatus 30 can inform the user about the undesired behavior.

In general, a detected possible disturbance can be either an actual disturbance of the EEG measurement, e.g., a disturbance of an EEG signal, or an increased likelihood of an imminent disturbance. The apparatus 30 may detect an emerging disturbance even if the EEG signal of the EEG measurement is not now disturbed. For example, the processing circuitry 34 may detect a possible disturbance by comparing a predefined parameter with data from the received input signal or a parameter determined based on the received input signal. For example, a measured pose of the head of the user can be compared with a predefined pose. If the measured pose deviates from the predefined pose by a certain pose threshold, the EEG measurement may be disturbed. For example, data about an acceleration of the head of the user can be compared with a predefined movement threshold. If the movement threshold is exceeded the EEG measurement may be disturbed.

For example, the detected possible disturbance may indicate that a likelihood for disturbing the EEG signal may be increased. By triggering the output the user can be informed about the increased likelihood of a disturbance of the EEG signal. Thus, the user can take certain measures to counteract the detected disturbance before the EEG signal is disturbed. This can reduce an overall time required for the EEG measurement by preventing interruption of the measurement of the EEG signal.

In an example, the processing circuitry 34 may be configured to detect the possible disturbance of the EEG measurement based on detecting an undesired behavior of the user. The user can be informed by the triggered output about the detected undesired behavior. In this way, the user can take measures to counteract the possible disturbance of the EEG measurement.

An undesired behavior of the user can be any behavior and/or result of a behavior which could disturb the EEG measurement, e.g., causing electrical contaminants in the EEG signal and/or decreasing a contact of the one or more sensor electrode to the head. In an example, an undesired behavior of the user is at least one of a head movement, an eye movement, shivering, frequent eye blinking, speaking, an inappropriate muscle activity (e.g., chewing, frowning etc) or an undesired position of the head of the user (e.g., a pose of the head). By detecting a type of undesired user behavior, the user can be informed about the kind of behavior. For example, the detected irregularity can be transformed in an output that the user understands (e.g., pop-up message provided by an application). Thus, the user can take measures to counteract the concrete behavior.

For example, an undesired behavior can be detected by the processing circuitry 34 based on data of one or more sensors. Optionally, a plurality of undesired behaviors can be detected by the processing circuitry 34 based on the data of one or more sensors. For example, an output signal of a camera can be used to detect an eye movement and frequent eye blinking. Thus, detecting a possible disturbance of the EEG measurement can be based different information user behaviors. By considering different user behaviors a detection of the possible disturbance can be improved.

For example, the detection of a possible disturbance can be based on a plurality of undesired behaviors. For example, if a first behavior, e.g., an eye movement, exceeds a first predefined eye threshold and a second behavior, e.g., a blink rate, does not exceed a second predefined blink threshold the EEG measurement can be assumed as undisturbed. Alternatively, even if only one behavior is below or above a predefined threshold, the EEG measurement may be assumed as being disturbed due to the user behavior. In this way, a possible disturbance can be detected based on a combination of multiple detected behaviors.

In an example, the interface circuitry 32 may be configured to receive a measurement signal comprising information about an EEG signal measured by one or more sensor electrodes of an EEG device (e.g., from a head-mountable measurement device or an EEG cap). The measurement signal may be part of the input signal or an additional input signal. The measurement signal may comprise or may be an EEG signal. Alternatively, the measurement signal may comprise raw data generated by the one or more sensor electrodes. For example, the processing circuitry 34 may be configured to determine the EEG signal based on the measurement signal.

For example, the processing circuitry 34 may be configured to detect a possible disturbance of the EEG measurement based on the information about the EEG signal. For example, the EEG signal may show an irregularity which may correlate to an undesired user behavior. Thus, the EEG measurement can be assumed to be disturbed by the user behavior. In this way, a reliability of the detection of a possible disturbance caused by the user behavior can be increased.

An output to inform the user can be triggered when a possible disturbance of the EEG signal, e.g., an electrical contamination in the EEG signal, correlates to an undesired user behavior. For example, the output can be triggered when a movement of the head exceeds a movement threshold, and the EEG signal is disturbed or is likely to become disturbed. By correlating the possible EEG signal with the undesired user behavior, the user can be informed about the undesired behavior.

For example, the user may show multiple undesired behaviors. However, only one or few of these multiple undesired behaviors may cause a possible disturbance of the EEG measurement. In this case, the user can be informed about the concrete undesired behavior causing the possible disturbance. In this way, the user can take measures to counteract the concrete undesired behavior.

Additionally, the user can be informed by the output about the disturbed EEG signal. For example, the interface circuitry 32 or an output circuitry can transmit an output signal indicative of the disturbed EEG signal. The disturbed EEG signal may comprise an irregularity, e.g., caused by electrical contamination. The disturbed EEG signal can be displayed on the user display. By displaying the disturbed EEG signal the user can be informed about the disturbance of the EEG measurement, e.g., a time of the disturbance, a strength of the disturbance. Thus, the user can take certain measures to reduce the strength of the disturbance and/or the duration of the disturbance.

For example, a real-time information about the EEG signal may be shown on the user display. When the EEG signal is disturbed, a disturbed area of the EEG signal can be highlighted. By highlighting the area in real-time the user can receive a real-time feedback about the disturbance of the EEG signal, e.g., the strength of the disturbance. In this way, the user can take certain measures to counteract the disturbance of the EEG signal. For example, the real-time information about the EEG signal may be combined with the information about the undesired behavior. By combining the EEG signal and the information about the undesired behavior, the user can counteract the undesired behavior until the EEG signal is no longer disturbed. In this way, the user can take more precise measures to improve the EEG measurement.

In an example, the input signal may comprise information about a movement of a head-mountable measurement device comprising one or more electrodes. Further, the processing circuitry 34 may be configured to determine whether the movement is higher than or equal to a movement threshold. When the movement is higher than or equal to the movement threshold a possible disturbance of the EEG measurement may be detected. By detecting the movement of the head-mountable measurement device, a possible disturbance of the EEG measurement can be detected. The movement can comprise a movement of the head-mountable measurement device, e.g., a maximal amplitude of the movement in a spatial direction, an acceleration and/or a movement speed of the head-mountable measurement device.

In an example, the processing circuitry 34 may be further configured to at least one of trigger an interruption of the EEG measurement, correct a disturbed part of the EEG measurement or mark a disturbed part of the EEG measurement based on the detected possible disturbance. By triggering an interruption of the EEG measurement a measurement of a disturbed EEG signal can be avoided. In this way, a data acquisition can be reduced. By correcting a disturbed part of the EEG measurement, the EEG measurement can still be used for further evaluation. In this way, a repetition of the EEG measurement can be avoided. By marking a disturbed part of the EEG measurement, e.g., a disturbed part of the EEG signal, information provided to the user or stored for later analysis can be improved (as described above). In this way, the user can take more precise measures to counteract a possible disturbance of the EEG measurement.

For example, a movement of the head-mountable measurement device may be higher than a threshold and thus the EEG measurement may be interrupted. Optionally, the interruption can be terminated when the movement of the EEG cap is again below the threshold. Thus, the EEG measurement can be continued when the undesired behavior has disappeared or is no longer significant. In an example, the processing circuitry 34 may be further configured to resume the EEG measurement after an interruption if the possible disturbance or the user behavior causing the possible disturbance has vanished or is below a threshold. In this way, a complete repetition of the EEG measurement or a postprocessing of a disturbed EEG signal can be avoided.

As described above, the possible disturbance can be detected based on an undesired behavior and optionally on an (disturbed) EEG signal. Thus, resuming the EEG measurement can be also based on the undesired behavior and optionally on the EEG signal. For example, the EEG measurement was interrupted since an undesired behavior exceeds an upper threshold, e.g., an upper movement threshold, causing a disturbed EEG signal. Further, an output was triggered to inform the user about the disturbed EEG signal. Based on the output the user counteracted the undesired behavior. Thus, the undesired behavior can be again below the upper threshold and the EEG signal may no longer show a disturbance. Therefore, the EEG measurements can be resumed. In this way, the EEG measurement can be quickly resumed without having to eliminate the undesired behavior completely.

However, the user may still show an undesired behavior that has been reduced just enough to continue the EEG measurement. Accordingly, the output can still be triggered even if the EEG measurement was resumed. For example, the output can be triggered until the undesired behavior is below a lower threshold (e.g., a maximum speed or acceleration of the head-mountable measurement device or a degree of jaw contraction is below the upper threshold). The lower threshold may indicate that the undesired behavior has vanished. In this way, the user can be informed about the undesired behavior until the undesired behavior vanishes.

Alternatively, the EEG measurement can be resumed when the undesired behavior has vanished, e.g., below the lower threshold. In this way, the EEG measurement can be resumed with a decreased likelihood of a disturbance of the EEG measurement.

In an example, the output to the user is at least one of a visual output, an acoustic output or a tactile output. For example, the output can be a visual, an audible, a vibrating or a tactile output, or a combination thereof. For example, the user can be informed by an output on a display of the user device, by a sound of a loudspeaker of the user device or the head-mountable measurement device, by a vibration of the user device or the head-mountable measurement device.

The output to the user can be provided by an application associated with the EEG measurement, e.g., used to control or run the EEG measurement. For example, the application can be shut down or frozen, can output information or an input to operate the application can be blocked. In this way, the user can be informed in an eased way by the application used to control the EEG measurement. For example, the user may interact with the user device (e.g., the user may play a game) while an EEG measurement is performed. If a possible disturbance of the EEG measurement is detected, the interaction can be interrupted, a message can pop up and/or a red screen can be shown, for example, to inform the user on the possible disturbance.

For example, the user can perform certain tasks in the application running on the user device. For example, the tasks may be designed to trigger specific brain regions, distract the attention of the user from the EEG measurement and/or to entertain the user during the EEG measurement. By drawing the attention of the user away from the EEG measurement, especially by focusing on a different task, an undesired behavior may be reduced or even avoided. For example, an undesired movement of the head of the user can be counteracted by drawing the attention of the user to the display of the user device. In this way, the quality and/or accuracy of the EEG measurement may be improved.

Optionally, the information provided to the user can be associated with the task preformed in the application. For example, the user may receive a positive feedback when a task, e.g., bringing trained parameters in a direction of interest, is completed. When a possible disturbance of the EEG measurement is detected, the user may no longer receive a positive feedback independently of task completion. By disabling the positive feedback the user can take measures to regain the positive feedback. In this way, the user may counteract the possible disturbance of the EEG measurement to regain the positive feedback.

For example, the application may be a game that attracts the attention of the user. The positive feedback can be a progress in the game. For example, the game may be frozen, or the positive feedback may be disabled when an undesired behavior is detected. Thus, the user can only play/progress in the game when the EEG measurement is undisturbed. In this way, the user may counteract the possible disturbance of the EEG measurement to play the game. For example, the user may be a child or any other person.

In an example, the input signal may further comprise information indicative of the behavior of a user based on output signals of a plurality of sensors. Further, the processing circuitry 34 may be configured to detect a possible disturbance of the EEG measurement based on the output signals of the plurality of sensors. By combining information from multiple sensors a user behavior that is not directly visible can be detected. Gritting teeth of the user can be determined by a camera and an acceleration sensor, for example. In this way, a detection of the user behavior can be improved.

As shown in Fig. 1, the interface circuitry 32 is coupled to the processing circuitry 34 at the apparatus 30. In examples, the processing circuitry 34 may be implemented by one or more processing units, one or more processing devices, any means for processing, such as a processor, a computer or a programmable hardware component being operable with accordingly adapted software. Similar, the described functions of the processing circuitry 34 may as well be implemented in software, which is then executed on one or more programmable hardware components. Such hardware components may comprise a general-purpose processor, a Digital Signal Processor (DSP), a micro-controller, etc. The processing circuitry 34 may be capable of controlling the interface circuitry 32, so that any data transfer that occurs over the interface circuitry 32 and/or any interaction in which the interface circuitry 32 may be involved may be controlled by the processing circuitry 34.

In an embodiment the apparatus 30 may comprise a memory and at least one processing circuitry 34 operably coupled to the memory and configured to perform the functionalities described above or below.

In examples the interface circuitry 32 may correspond to any means for obtaining, receiving, transmitting or providing analog or digital signals or information, e.g., any connector, contact, pin, register, input port, output port, conductor, lane, etc. which allows providing or obtaining a signal or information. The interface circuitry 32 may be wireless or wireline and it may be configured to communicate, e.g., transmit or receive signals, information with further internal or external components.

The apparatus 30 may be or may be part of a computer, processor, control unit, (field) programmable logic array ((F)PLA), (field) programmable gate array ((F)PGA), graphics processor unit (GPU), application-specific integrated circuit (ASICs), integrated circuits (IC) or system-on-a-chip (SoCs) system.

The apparatus 30 may be or may be part of an EEG device or may be connected to an EEG device. The EEG device may be a head-mountable measurement device or may be connectable to a head-mountable measurement device. The EEG device may be part of an EEG system comprising the EG device and a user device. The user device may be a tablet, a smart phone, a laptop or a computer. The user device may be configured to control the EEG device and/or show the user an output of the EEG device and/or run an application for interaction with the user during an EEG measurement.

By measuring brain waves with an EEG device and visualizing them in an application, children can learn to control their brain activity. The proposed EEG device may enable a home-based neurofeedback therapy that allows the user to playfully train his brain to help alleviate and/or treat symptoms.

More details and aspects are mentioned in connection with the examples described below. The example shown in Fig. 1 may comprise one or more optional additional features corresponding to one or more aspects mentioned in connection with the proposed concept or one or more examples described below (e.g., Fig. 2 - 8).

Fig. 2 shows an example of a block diagram of an apparatus 50 for controlling an EEG measurement. The apparatus 50 comprises interface circuitry 52 and processing circuitry 54. The interface circuitry 52 is configured to receive an input signal comprising information indicative of a humidity at a head of a user. The input signal is based on an output signal of a humidity sensor of an EEG device. Further, the processing circuitry 54 is configured to determine whether the humidity at the head of the user exceeds a humidity threshold that affects the EEG measurement. Further, the processing circuitry is configured to trigger an output to the user to inform the user about a possible disturbance of the EEG measurement. By using sensor data of the humidity sensor an effect on the EEG measurement can be determined. For example, the effect may disturb the EEG measurement, e.g., may cause a disturbed EEG signal, a decreased strength of the EEG signal. By informing the user about the effect, the user can take certain measures to counteract the effect. In this way, an EEG measurement can be controlled in an improved way.

The humidity at the head may influence the EEG measurement. For example, a contact between a sensor electrode and the head of the user may be influenced by the humidity at the head of the user. Variations of the contact of the sensor electrode on the head may result instabilities in the impedance and induced potentials at the sensor electrode-scalp interface. Furthermore, salt bridges can form between adjacent electrodes, resulting in a short-circuit and a signal recorded by the involved electrodes that does not reflect the level of brain waves. This can produce artifacts, which may have an effect on the EEG measurement. Thus, by comparing the humidity with a humidity threshold an effect on the EEG measurement can be determined.

As described with reference to Fig. 1 the threshold, e.g., the humidity threshold, can be either a threshold indicating an increased likelihood of disturbance of the EEG measurement (lower humidity threshold) or indicating a disturbed EEG signal (higher humidity threshold). In this way, the user can be informed about different effects on the EEG measurements triggered by different thresholds.

For example, when a lower humidity threshold is exceeded, the user can be informed by a first output about an increased likelihood of a disturbed EEG measurement, e.g., due to sweating. When a higher humidity threshold is exceeded, the user can be informed in a second output after the first output about a disturbed EEG signal, e.g., due to excessive sweating. In this way, the user can take precise measures to counteract the effect on the EEG measurement.

The apparatus 50 can be implemented similar to the apparatus as the apparatus described with reference to Fig. 1. For example, the apparatus 50 may comprise one or more features of the apparatus described with reference to Fig. 1.

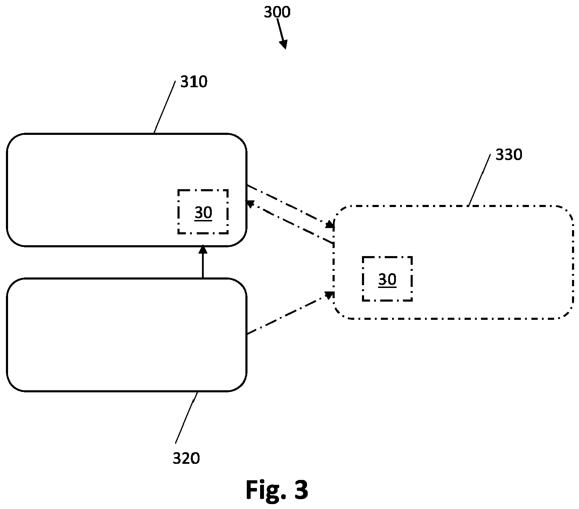

More details and aspects are mentioned in connection with the examples described above and/or below. The example shown in Fig. 2 may comprise one or more optional additional features corresponding to one or more aspects mentioned in connection with the proposed concept or one or more examples described above (e.g., Fig. 1) and/or below (e.g., Fig. 3 - 8).

Fig. 3 shows an example of an EEG system 300 comprising an apparatus 30 as described in Fig.1 or Fig. 2. The EEG system 300 comprises an EEG device 310, an apparatus 30 as described above, e.g., with reference to Fig. 1 or Fig. 2 and a sensor 320 configured to generate the output signal. For example, the EEG system 300 may also comprise a user device 330, e.g., as described in more detail with reference to Fig. 1. The apparatus 30 may part of either the EEG device 310 or the user device 330.

An application to run an EEG measurement can be installed on the user device 330. The user device 330 may be communicatively coupled to the EEG device 310, e.g., via Bluetooth or wireless local area network.

The EEG device 310 comprises multiple sensor electrodes that make contact with the head of the user, especially the scalp and/or skin. Additionally the EEG device 310 comprises interface circuitry communicatively coupled to the multiple sensors to provide electrical signals received from the multiple sensor electrodes. The electrical signals may be combined to an EEG signal. The interface circuitry may be communicatively coupled to an interface circuitry of the apparatus 30. Thus, the apparatus 30 can receive the measurement signal from the interface circuitry of the EEG device 310. The measurement signal may comprise the EEG signal or data (e.g., raw data) indicative of the EEG signal from the multiple sensor electrodes. When the measurement signal comprises data indicative of the EEG signal, the apparatus may be configured to determine the EEG signal.

In an example, the sensor may be at least one of an accelerometer, a gyroscope, an additional electrode, a camera, a humidity sensor or a microphone. By implementing the sensor along with the EEG device 310 or the user device 330, while also using the user device 330 for an application correlated to an EEG measurement, a feedback to the user can be provided when an undesired behavior of the user is detected, e.g., a muscle activity or a movement.

For example, the user may use the user device 330 to run an EEG measurement using the EEG device 310. The user device 330 may be communicatively coupled to the EEG device 310 to receive data from the EEG system 300, e.g., the measurement signal. The sensor 320 may part of the EEG device 310 or may be part of the user device 330. The user device 330 may receive the measurement signal and/or the output signal from the sensor 320.

For example, the output signal may be processed by the user device 330, e.g., the application to control or run the EEG measurement. If a threshold is exceeded, e.g., an acceleration of the head of the user, the application may stop the EEG measurement by triggering an output to the EEG device 310 to stop the EEG measurement. Further, the application on the user device 330 may inform the user about a possible disturbance of the EEG measurement. In this way, a medically certified home-based neurofeedback therapy can be performed by the user, where no specialized staff is necessary to monitor the user.

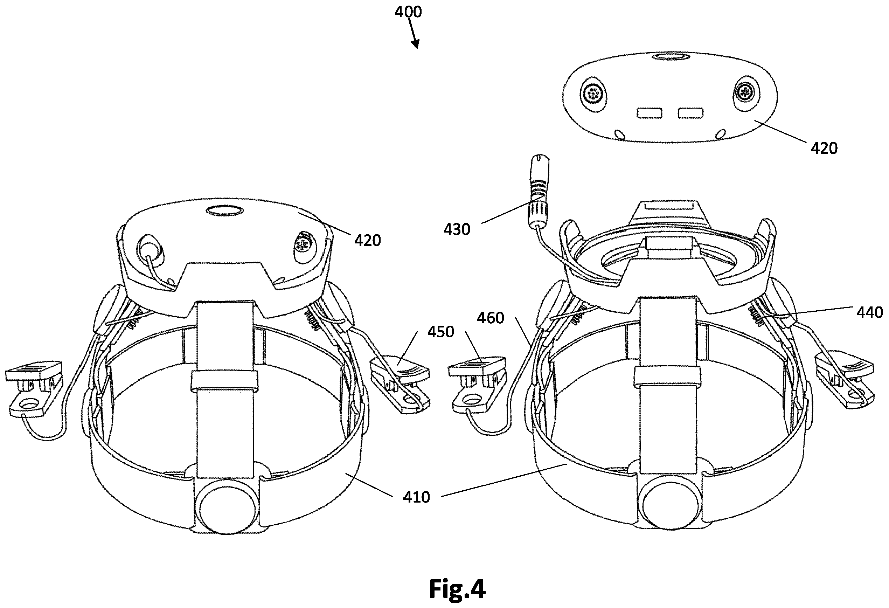

More details and aspects are mentioned in connection with the examples described above and/or below. The example shown in Fig. 3 may comprise one or more optional additional features corresponding to one or more aspects mentioned in connection with the proposed concept or one or more examples described above (e.g., Fig. 1 - 2) and/or below (e.g., Fig. 4 - 8).

An EEG system may be implemented as a single device or may comprise several components. For example, the EEG system may comprise a head-mountable measurement device and a processing device.

For example, a head-mountable measurement device comprises a mounting structure configured to fasten the head-mountable measurement device to a head of a user. Further, the head-mountable measurement device comprises a plurality of contact electrodes configured to contact the head of the user. Additionally, the head-mountable measurement device comprises interface circuitry electrically connected to the plurality of contact electrodes. The interface circuitry is connected to provide electrical signals received from the plurality of contact electrodes to a processing device. The head-mountable measurement device further comprises a position adjustment structure configured to enable an individual adjustment of a position of at least one contact electrode of the plurality of contact electrodes (e.g., independent from the other contact electrodes).

The head-mountable measurement device may comprise the processing device or may be connectable to the processing device. For example, the interface circuitry may be an interface to be connected to a removable processing device. The processing device may comprise or may be an apparatus for controlling an electroencephalography, EEG, measurement or an EEG device as described above or below.

The mounting structure may comprise a headband. The head-mountable measurement device may also be called EEG cap, EEG headset, EEG headband or EEG hat.

Fig. 4 shows different views of an example of an EEG device 400 comprising an EEG cap 410 and a control device 420. The control device 420 can be mounted to the EEG cap 410. Further, the control device 420 can be communicatively coupled via a connector 430 with the EEG cap 410, especially with sensor electrodes 440. The EEG cap 410 may comprise multiple sensor electrodes 440 used to measure the brain activity. Further, the EEG cap may comprise ear clips 450 used as a reference for the sensor electrode 440. The ear clips 450 can be clipped on the earlobes. The electrode cable may connect the sensor electrodes 440 and may comprise the ear clips 450.

The apparatus for controlling an electroencephalography measurement as described above (e.g., in connection with Fig. 1 or 2) can be part of the EEG cap 410 or of the control device 420. The sensor for measuring a parameter indicating the user behavior can be part of the EEG cap 410 or the control device 420. For example, the EEG device 400 may trigger the output to the user by transmitting an output signal to a user device. Alternatively, the EEG device 400 may comprise an output device, such as a loudspeaker and can trigger the output by use of the loudspeaker.

Alternatively, neither the apparatus nor the sensor may be part of the EEG device 400. In this case the EEG device 400 may only transmit a measurement signal indicative of the EEG signal of the EEG measurement to the apparatus for controlling an electroencephalography measurement and/or the user device.

The sensor (not shown), e.g., a gyroscope, accelerometer, may be part of the control device 420. In this way, data transfer between the apparatus and the sensor can be eased. For example, the sensor and the apparatus are arranged on or connected to the same printed circuit board of the control device 420.

As described above the apparatus for controlling an electroencephalography measurement may be part of the control device 420. Thus, the control device 420 can determine the disturbance of the EEG measurement. Based on the determined disturbance an output to the user device can be triggered. In this way, a data transfer between the control device 420 and the user device can be decreased.

Alternatively, the apparatus may be comprised by the user device (not shown). Thus, the computational task to determine the disturbance of the EEG measurement can be moved to the user device. In this way, a needed computational resource of the control device 420 can be decreased.

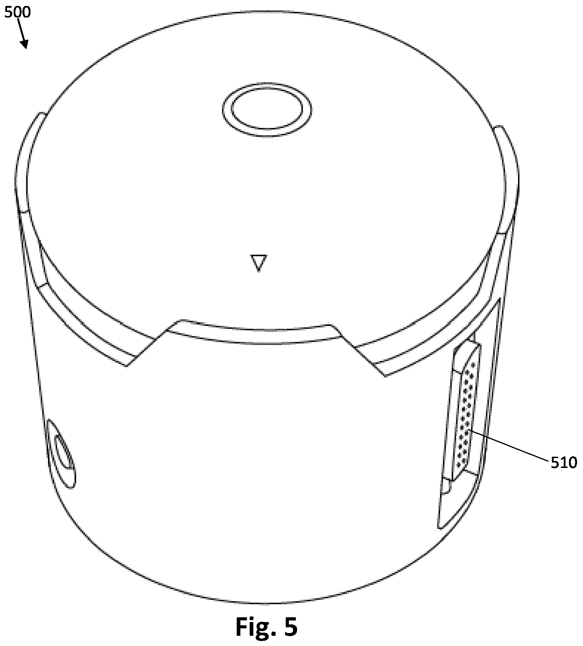

More details and aspects are mentioned in connection with the examples described above and/or below. The example shown in Fig. 4 may comprise one or more optional additional features corresponding to one or more aspects mentioned in connection with the proposed concept or one or more examples described above (e.g., Fig. 1 - 3) and/or below (e.g., Fig. 5 - 8).

Fig. 5 shows a perspective view of a base station 500. The base station 500 may comprise the apparatus for controlling an electroencephalography measurement as described above. The base station may be an alternative concept to the control device as described in Fig. 4. The base station 500 may comprise the sensor, e.g., a camera, to determine the user behavior. The base station 500 may be communicatively coupled to the user device, e.g., to trigger an output to inform the user about a disturbance of the EEG measurement.

An EEG cap can be connected to the base station 500 via the connector 510. The base station 500 can determine the disturbance of the EEG measurement. In this way, a data traffic between the base station 500 and the user device can be reduced. Alternatively, the base station may transmit the information needed to determine the disturbance of the EEG measurement to the user device. Thus, the user device can determine the disturbance of the EEG measurement. In this way, needed computational resources of the base station 500 can be reduced.

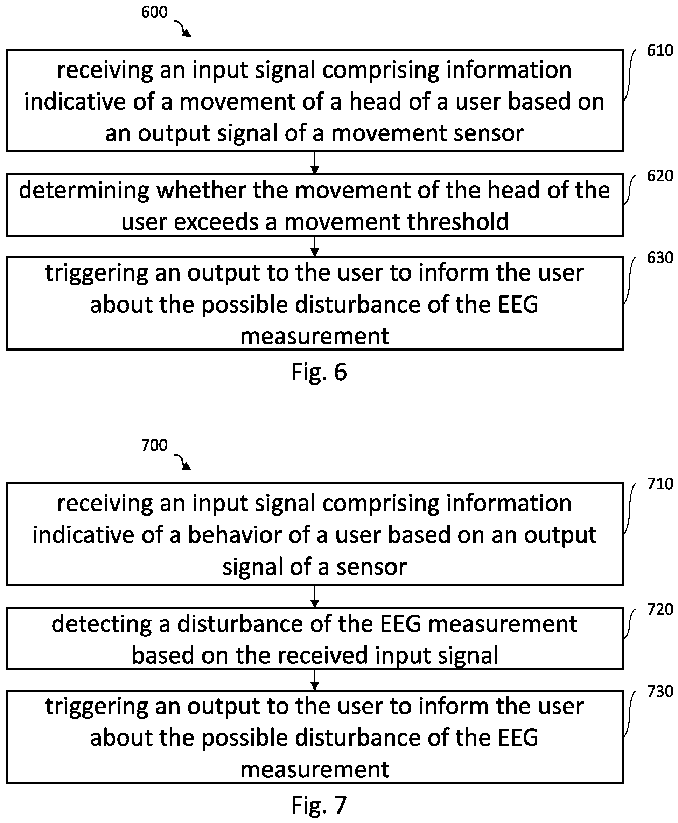

More details and aspects are mentioned in connection with the examples described above and/or below. The example shown in Fig. 5 may comprise one or more optional additional features corresponding to one or more aspects mentioned in connection with the proposed concept or one or more examples described above (e.g., Fig. 1 - 4) and/or below (e.g., Fig. 6 - 8).

Fig. 6 shows an example of a method 600 for controlling an EEG measurement. The method 600 comprises receiving 610 an input signal comprising information indicative of a movement of a head of a user based on an output signal of a movement sensor. Further, the method 600 comprises determining 620 whether the movement of the head of the user exceeds a movement threshold. Further, the method 600 comprises triggering 630 an output to the user to inform the user about the possible disturbance of the EEG measurement. The method can be performed by an apparatus as described above, e.g., in Fig. 1 or 2.

More details and aspects are mentioned in connection with the examples described above and/or below. The example shown in Fig. 6 may comprise one or more optional additional features corresponding to one or more aspects mentioned in connection with the proposed concept or one or more examples described above (e.g., Fig. 1 - 5) and/or below (e.g., Fig. 7 - 8).

Fig. 7 shows another example of a method 700 for controlling an EEG measurement. The method 700 comprises receiving 710 an input signal comprising information indicative of a behavior of a user based on an output signal of a sensor. Further, the method 700 comprises detecting 720 a disturbance of the EEG measurement based on the received input signal. The method 700 also comprises triggering 730 an output to the user to inform the user about the possible disturbance of the EEG measurement. The method can be performed by an apparatus as described above, e.g., in Fig. 1 or 2.

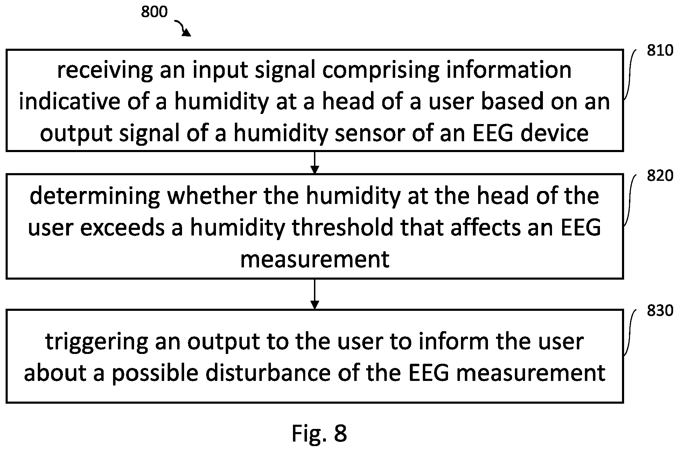

More details and aspects are mentioned in connection with the examples described above and/or below. The example shown in Fig. 7 may comprise one or more optional additional features corresponding to one or more aspects mentioned in connection with the proposed concept or one or more examples described above (e.g., Fig. 1 - 6) and/or below (e.g., Fig. 8).

Fig. 8 shows another example of a method 800 for controlling an EEG measurement. The method 800 comprises receiving 810 an input signal comprising information indicative of a humidity at a head of a user. The input signal is based on an output signal of a humidity sensor of an EEG device. Further, the method 800 comprises determining 820 whether the humidity at the head of the user exceeds a humidity threshold that affects an EEG measurement. The method 800 also comprises triggering 830 an output to the user to inform the user about a possible disturbance of the EEG measurement. The method can be performed by an apparatus as described above, e.g., in Fig. 1 or 2.

More details and aspects are mentioned in connection with the examples described above. The example shown in Fig. 8 may comprise one or more optional additional features corresponding to one or more aspects mentioned in connection with the proposed concept or one or more examples described above (e.g., Fig. 1 - 7).

The aspects and features described in relation to a particular one of the previous examples may also be combined with one or more of the further examples to replace an identical or similar feature of that further example or to additionally introduce the features into the further example.

Examples may further be or relate to a (computer) program including a program code to execute one or more of the above methods when the program is executed on a computer, processor or other programmable hardware component. Thus, steps, operations or processes of different ones of the methods described above may also be executed by programmed computers, processors or other programmable hardware components. Examples may also cover program storage devices, such as digital data storage media, which are machine-, processor- or computer-readable and encode and/or contain machine-executable, processor-executable or computer-executable programs and instructions. Program storage devices may include or be digital storage devices, magnetic storage media such as magnetic disks and magnetic tapes, hard disk drives, or optically readable digital data storage media, for example. Other examples may also include computers, processors, control units, (field) programmable logic arrays ((F)PLAs), (field) programmable gate arrays ((F)PGAs), graphics processor units (GPU), application-specific integrated circuits (ASICs), integrated circuits (ICs) or system-on-a-chip (SoCs) systems programmed to execute the steps of the methods described above.

It is further understood that the disclosure of several steps, processes, operations or functions disclosed in the description or claims shall not be construed to imply that these operations are necessarily dependent on the order described, unless explicitly stated in the individual case or necessary for technical reasons. Therefore, the previous description does not limit the execution of several steps or functions to a certain order. Furthermore, in further examples, a single step, function, process or operation may include and/or be broken up into several sub-steps, - functions, -processes or -operations.

The computer-executable instructions can be part of, for example, an operating system of the computing system, an application stored locally to the computing system, or a remote application accessible to the computing system (e.g., via a web browser). Any of the methods described herein can be performed by computer-executable instructions performed by a single computing system or by one or more networked computing systems operating in a network environment. Computer-executable instructions and updates to the computer-executable instructions can be downloaded to a computing system from a remote server.

As used in this application and the claims, a list of items joined by the term "and/or" can mean any combination of the listed items. For example, the phrase "A, B and/or C" can mean A; B; C; A and B; A and C; B and C; or A, B and C. As used in this application and the claims, a list of items joined by the term "at least one of' can mean any combination of the listed terms. For example, the phrase "at least one of A, B or C" can mean A; B; C; A and B; A and C; B and C; or A, B, and C. Moreover, as used in this application and the claims, a list of items joined by the term "one or more of' can mean any combination of the listed terms. For example, the phrase "one or more of A, B and C" can mean A; B; C; A and B; A and C; B and C; or A, B, and C.

The disclosed methods, apparatuses, and systems are not to be construed as limiting in any way. Instead, the present disclosure is directed toward all novel and nonobvious features and aspects of the various disclosed embodiments, alone and in various combinations and sub combinations with one another. The disclosed methods, apparatuses, and systems are not limited to any specific aspect or feature or combination thereof, nor do the disclosed embodiments require that any one or more specific advantages be present or problems be solved.

Theories of operation, scientific principles, or other theoretical descriptions presented herein in reference to the apparatuses or methods of this disclosure have been provided for the purposes of better understanding and are not intended to be limiting in scope. The apparatuses and methods in the appended claims are not limited to those apparatuses and methods that function in the manner described by such theories of operation.

Although the operations of some of the disclosed methods are described in a particular, sequential order for convenient presentation, it is to be understood that this manner of description encompasses rearrangement, unless a particular ordering is required by specific language set forth herein. For example, operations described sequentially may in some cases be rearranged or performed concurrently. Moreover, for the sake of simplicity, the attached figures may not show the various ways in which the disclosed methods can be used in conjunction with other methods.

If some aspects have been described in relation to a device or system, these aspects should also be understood as a description of the corresponding method. For example, a block, device or functional aspect of the device or system may correspond to a feature, such as a method step, of the corresponding method. Accordingly, aspects described in relation to a method shall also be understood as a description of a corresponding block, a corresponding element, a property or a functional feature of a corresponding device or a corresponding system.

As used herein, the term "module" refers to logic that may be implemented in a hardware component or device, software or firmware running on a processing unit, or a combination thereof, to perform one or more operations consistent with the present disclosure. Software and firmware may be embodied as instructions and/or data stored on non-transitory computer-readable storage media. As used herein, the term "circuitry" can comprise, singly or in any combination, non-programmable (hardwired) circuitry, programmable circuitry such as processing units, state machine circuitry, and/or firmware that stores instructions executable by programmable circuitry. Modules described herein may, collectively or individually, be embodied as circuitry that forms a part of a computing system. Thus, any of the modules can be implemented as circuitry. A computing system referred to as being programmed to perform a method can be programmed to perform the method via software, hardware, firmware, or combinations thereof.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

## Claims

1. An apparatus (30) for controlling an electroencephalography, EEG, measurement, comprising:
interface circuitry (32) configured to receive an input signal comprising information indicative of a behavior of a user based on an output signal of a sensor; and
processing circuitry (34) configured to:
detect a possible disturbance of the EEG measurement based on the received input signal; and
trigger an output to the user to inform the user about the possible disturbance of the EEG measurement.

2. The apparatus (30) according to claim 1, wherein the processing circuitry (34) is further configured to
detect the possible disturbance of the EEG measurement based on detecting an undesired behavior of the user.

3. The apparatus (30) according to claim 2, wherein
an undesired behavior of the user is at least one of a head movement, an eye movement, shivering, frequent eye blinking, an inappropriate muscle activity or a position of the head of the user.

4. The apparatus (30) according to any one of the preceding claims, wherein the interface circuitry (32) is configured to
receive a measurement signal comprising information about an EEG signal measured by one or more sensor electrodes of an EEG device (310; 400).

5. The apparatus (30) according to any one of the preceding claims, wherein
the input signal comprises information about a movement of the EEG device (310; 400), wherein the processing circuitry (34) is configured to
determine whether the movement is higher than a movement threshold to detect a possible disturbance of the EEG measurement.

6. The apparatus (30) according to any one of the preceding claims, wherein the processing circuitry (34) is further configured to
at least one of trigger an interruption of the EEG measurement, correct a disturbed part of the EEG measurement or mark a disturbed part of the EEG measurement based on the detected possible disturbance.

7. The apparatus (30) according to claim 6, wherein the processing circuitry (34) is further configured to
resume the EEG measurement after an interruption if the possible disturbance has vanished or is below a threshold.

8. The apparatus (30) according to any one of the preceding claims, wherein
the output to the user is at least one of a visual output, an acoustic output or a tactile output.

9. The apparatus (30) according to any of the preceding claims, wherein
the input signal comprises information indicative of the behavior of a user based on output signals of a plurality of sensors; and wherein the processing circuitry (34) is further configured to
detect the possible disturbance of the EEG measurement based on the output signals of the plurality of sensors.

10. An apparatus (50) for controlling an electroencephalography, EEG, measurement, comprising:
interface circuitry (52) configured to receive an input signal comprising information indicative of a humidity at a head of a user based on an output signal of a humidity sensor of an EEG device (310; 400); and
processing circuitry (54) configured to:
determine whether the humidity at the head of the user exceeds a humidity threshold that affects the EEG measurement; and
trigger an output to the user to inform the user about a possible disturbance of the EEG measurement.

11. An electroencephalography, EEG, device (310; 400), comprising:
an apparatus (30; 50) according to any of the preceding claims; and
a sensor configured to generate the output signal.

12. The EEG device (310; 400) according to claim 11, wherein
the sensor is at least one of an accelerometer, a gyroscope, a camera, a humidity sensor or a microphone.

13. Method (600) for controlling an electroencephalography, EEG, measurement, comprising:
receiving (610) an input signal comprising information indicative of a movement of a head of a user based on an output signal of a movement sensor;
determining (620) whether the movement of the head of the user exceeds a movement threshold; and
triggering (630) an output to the user to inform the user about the possible disturbance of the EEG measurement.

14. Method (800) for controlling an electroencephalography, EEG, measurement, comprising:
receiving (810) an input signal comprising information indicative of a humidity at a head of a user based on an output signal of a humidity sensor of an EEG device;
determining (820) whether the humidity at the head of the user exceeds a humidity threshold that affects an EEG measurement; and
triggering (830) an output to the user to inform the user about a possible disturbance of the EEG measurement.

15. A computer program having a program code for performing a method (600; 800) according to claim 13 or 14 when the program is executed on processor.
